# FASCICULE DE BREVET EUROPEEN

(11) **EP 1 646 877 B1**
(45) Date de publication et mention de la délivrance du brevet: **30.01.2019**
(21) Numéro de dépôt: 04767602.8
(22) Date de dépôt: 07.07.2004
(51) Int. Cl.: G01N 35/00, B01F 11/00, B01L 7/00, B01L 9/06, B01F 15/06

(54) **DISPOSITIF DE CONTROLE DE QUALITE POUR UN ANALYSEUR SANGUIN FONCTIONNANT EN SANG TOTAL**
QUALITÄTSKONTROLLVORRICHTUNG FÜR EIN BLUTANALYSEGERÄT UNTER VERWENDUNG VON VOLLBLUT
QUALITY CONTROL DEVICE FOR A BLOOD ANALYSER USING WHOLE BLOOD

(30) Priorité: 21.07.2003 FR 0308863
(43) Date de publication de la demande: 19.04.2006
(73) Titulaire: Horiba ABX SA., 34184 Montpellier Cedex 4 (FR)
(72) Inventeur: LE COMTE, Roger, F-34470 Perols (FR)
(74) Mandataire: Plaçais, Jean Yves
(86) Numéro de dépôt international: PCT/FR2004/001767
(87) Numéro de publication internationale: WO 2005/019835

(56) Documents cités:
- EP-A- 0 634 660
- EP-A- 0 726 453
- EP-A- 1 174 717
- WO-A-01/36982
- WO-A-02/26386
- US-A- 5 578 268
- US-A- 5 646 046
- US-A1- 2002 118 594
- US-A1- 2003 029 254
- US-A1- 2003 054 542

## Description

L'invention se rapporte aux analyseurs hématologique destinés à analyser automatiquement des échantillons de produits sanguins.

Elle concerne plus particulièrement un dispositif de contrôle de qualité pour un analyseur sanguin fonctionnant en sang total.

Dans ce qui suit, on entend par "analyseur" tout appareil capable d'effectuer des analyses à partir d'un tube de sang, de sérum, de plasma ou d'urine.

Par l'expression "analyseur sur sang total" on entend un analyseur effectuant des analyses sur du sang total, c'est à dire contenant tous les éléments du sang, en opposition avec les analyseurs fonctionnant sur plasma ou sérum.

Par l'expression "contrôle de qualité" on entend une procédure qui consiste à vérifier au minimum quotidiennement le bon fonctionnement de l'analyseur avant d'effectuer les examens des patients.

Par l'expression "sang de contrôle bas, haut ou normal" on entend un sang dont les valeurs sont choisies pour être plutôt faibles, élevées ou normales.

Les sangs de contrôle destinés aux analyseurs se présentent généralement dans un conditionnement de type "flacons" ou "tubes" et sont proposés avec des valeurs basses, normales et hautes afin de contrôler l'instrument sur l'ensemble de sa gamme de mesure.

Les règles de passage des sangs de contrôle dépendent des législations. Généralement il est nécessaire de passer au moins une fois par jour le niveau normal, plus un niveau bas ou haut. Dans certains cas, et notamment pour les appareils fonctionnant jour et nuit, il est nécessaire de passer au moins une fois le niveau normal au changement d'opérateur.

Si l'on se réfère à la norme NCCLS H38-P paragraphe 5.7.2, les auteurs font référence à l'importance majeure de l'agitation dans la méthode de contrôle de qualité, notamment concernant le temps d'agitation et la manière d'agiter les sangs de contrôle. Les recommandations officielles montrent qu'une meilleure maîtrise des outils pré-analytiques est très importante pour la pertinence du contrôle de qualité.

La conservation des sangs de contrôle se fait par le froid à la température recommandée par le fournisseur afin de garantir la date limite de péremption donnée par le fournisseur. Il doivent avoir repris la température ambiante et être soigneusement mélangés avant d'être passés sur l'analyseur. La remise en température est un facteur important dans le bon fonctionnement du contrôle de qualité.

Les sangs de contrôle sont livrés avec des feuilles de résultats donnant, pour chaque niveau de sang et pour chaque paramètre, la valeur cible à obtenir et les limites acceptables ou tolérances.

La date de péremption ainsi que le nombre maximum de prélèvements dans le même tube de sang de contrôle sont également donnés par le fabricant et doivent être soigneusement respectés.

Les résultats obtenus en analysant les sangs de contrôle doivent être archivés par le laboratoire. Ils sont souvent présentés sous la forme de graphes dits de "levey jennings" afin d'en faciliter l'interprétation.

La procédure de passage des sangs de contrôle dans un laboratoire consiste à mettre en route l'analyseur si nécessaire, à sortir les sangs de contrôle d'un réfrigérateur, à laisser les sangs sur une paillasse pendant quelques minutes pour qu'ils prennent la température ambiante, à agiter très soigneusement les sangs de contrôle, à effectuer les passages des sangs dans l'analyseur, à vérifier si les résultats sont dans les limites données par le fabricant et à remettre les sangs au réfrigérateur.

Dans ce processus il faut vérifier à chaque fois la date de péremption et respecter le nombre maximum d'utilisation successives du même tube ou flacon.

La même procédure est nécessaire pour l'utilisation d'un appareil fonctionnant jour et nuit dans un service d'urgence ou de soins intensifs ou encore dans le cadre de l'officine d'un médecin installé hors de l'hôpital obligeant l'opérateur à sortir les sangs du réfrigérateur et à effectuer l'ensemble de la procédure décrite ci dessus. Dans le cadre des utilisations ci-dessus les opérateurs sont souvent peu qualifiés et formés pour reproduire les procédures de contrôle réglementaires nécessaires au contrôle de l'analyseur.

Une phase importante de la procédure de contrôle de qualité est la remise en suspension du contrôle de qualité qui nécessite des dispositifs conçus pour effectuer une agitation régulière et non agressive des flacons.

On connaît en particulier, d'après US 2002/0118594A1, un dispositif d'agitation comportant un électro-aimant et une petite tige contenue dans le flacon. Le mouvement de l'électro-aimant fait bouger la tige contenue dans le flacon et provoque l'agitation sous la forme d'un vortex du sang à l'intérieur du flacon. Outre le fait que ce dispositif nécessite d'effectuer des opérations manuelles pour pouvoir être intégré dans une procédure de contrôle de qualité, le principe d'agitation qu'il met en oeuvre présente un certain nombre de risques vis-à-vis des cellules qui peuvent être détériorées lors de l'agitation.

On connaît aussi, d'après WO 08501797A1, un dispositif d'agitation par balancement qui équipe notamment les appareils automatiques de la société Beckman Coulter. Dans ce dispositif des cassettes contenant les tubes de contrôle sont chargées horizontalement sur un tapis roulant animé d'un mouvement de balancement qui permet l'agitation du sang.

On connaît également, d'après US 5 110 743 un dispositif d'agitation par retournement constitué d'un disque pouvant accueillir des tubes. Le disque est composé de deux sous-ensembles pouvant entrer en rotation indépendamment l'un de l'autre. Un disque est utilisé pour l'agitation et l'autre pour le stockage.

Les procédés d'agitation par balancement et par retournement peuvent être retenues comme principe d'agitation pour le contrôle de qualité. En aucun cas les brevets cités ci-dessus ne décrivent l'utilisation de ces principes d'agitation pour une fonction de contrôle de qualité intégrée telle que décrite dans la présente demande.

De nombreux brevets décrivent la composition de contrôle de qualité et mettent en avant des qualités de stabilité et de paramètres contrôlables. C'est le cas par exemple des brevets US 5 529 933, US 6 403 377, US 6 448 085. Cependant, aucun de ces brevets ne décrit de méthode d'agitation ou de procédure de passage des tubes de contrôle de qualité permettant de reproduire de manière optimum l'agitation et la conservation des échantillons du contrôle de qualité dans l'analyseur.

D'autres documents pertinents sont les brevets US 5 646 046, EP 0 726 453 et tout particulièrement le brevet US 2003/029254.

Etant donné l'importance du contrôle de qualité tant au plan législatif qu'au plan de la garantie de la qualité des analyses rendues aux patients, un des buts de l'invention est d'intégrer le contrôle de qualité dans les analyseurs et de l'automatiser totalement pour éliminer les erreurs liées aux manipulations. En étant totalement affranchi du contrôle de qualité, l'opérateur peut consacrer toute son attention aux résultats des patients.

La procédure de contrôle de qualité peut ainsi être prise en charge par l'analyseur qui l'exécute de manière automatique, seuls la fréquence des contrôles de qualité ainsi que le nombre et le choix des niveaux de sangs de contrôle étant programmables par l'opérateur.

Ce but est atteint par l'invention en proposant un dispositif de contrôle de qualité automatique intégré dans un analyseur sanguin fonctionnant en sang total, selon la revendication 1.

Pour effectuer un contrôle de qualité permettant de vérifier le bon fonctionnement de l'analyseur le dispositif selon l'invention comprend des moyens de conservation par refroidissement des sangs de contrôle, des moyens de remise en température des sangs de contrôle à la température prescrite par le fabricant des sangs de contrôle, des moyens d'agitation pour la remise en suspension des cellules et des moyens de prélèvement du sang ainsi préparé.

Selon une première caractéristique, les moyens de conservation du sang de contrôle comprennent un nombre déterminé de tubes fermés par un bouchon et rangés dans un support en contact avec un bloc de refroidissement permettant de réguler la température et de maintenir une température optimum pour la conservation du sang de contrôle.

Dans l'invention, le refroidissement des sangs de contrôle et leur remise en température constituent des opérations essentielles pour accomplir le contrôle de qualité dans des conditions optimales.

De façon avantageuse le bloc de refroidissement est un bloc de refroidissement à effet Peltier qui permet de contrôler la température du bloc à l'aide de moyens de régulation électronique. On rappellera qu'un tel bloc est alimenté en courant électrique et permet une régulation précise de la température. Il est en outre peu encombrant, ce qui facilite son intégration dans un analyseur.

Cependant l'invention ne se limite pas à l'utilisation d'un bloc de refroidissement à effet Peltier et d'autres types de bloc de refroidissement sont envisageables dans le cadre de l'invention.

Pour la remise en température, différentes solutions sont proposées.

Tout d'abord, et quel que soit le type de bloc de refroidissement utilisé, le support de tubes est désolidarisé du bloc de refroidissement pour la remise en température des sangs de contrôle.

Dans le cas d'un bloc de refroidissement à effet Peltier, selon un mode de réalisation ne faisant pas partie de la présente invention, le courant alimentant le bloc de refroidissement à effet Peltier peut être coupé pendant une période de temps déterminée afin de remettre en température les sangs de contrôle. L'arrêt du courant provoque une remontée de la température du bloc de refroidissement à la valeur ambiante.

Toujours dans le cas d'un bloc de refroidissement à effet Peltier, selon un mode de réalisation ne faisant pas partie de la présente invention, une autre solution consiste à commander pour remettre et maintenir le contrôle de qualité à sa température d'utilisation selon les spécifications du fabricant. On utilise alors l'effet Peltier en sens inverse pour provoquer un réchauffement, au lieu d'un refroidissement.

Les moyens d'agitation sont de préférence des moyens d'agitation par balancement et/ou retournement formés par le support de tubes, articulé autour d'une charnière du bloc de refroidissement. De préférence l'angle de retournement est compris entre 100° et 180°.

Selon une deuxième caractéristique, les moyens de prélèvement du sang sont formés par une aiguille propre à aspirer le sang dans les tubes. L'aiguille est animée d'un mouvement transversal au dessus des tubes de sang à analyser et des sangs de contrôle ainsi qu'au dessus de cuves de rinçage et de mélange et d'un mouvement vertical pour pénétrer dans les tubes par perçage des bouchons ou de descendre dans les cuves de rinçage et de mélange pour effectuer les rinçages ou les dilutions du sang.

Le perçage des bouchons s'effectue lorsque les tubes sur leur support sont en position haute et/ou basse.

Le dispositif comprend avantageusement des moyens de traitement programmables pour vérifier que les valeurs obtenues en passant chaque contrôle de qualité correspondent aux valeurs limites et attendues du sang de contrôle.

Les moyens de traitement déclenchent une alarme si les valeurs obtenues lors du passage du contrôle de qualité sont en dehors des limites attendues.

Le dispositif comprend en outre des moyens pour déclencher la procédure de contrôle de qualité soit directement par un opérateur ou de manière automatique ou par une connexion externe vers un organe de commande.

Le transfert et l'analyse des données s'effectuent par l'intermédiaire d'un réseau interne ou externe mettant en oeuvre les normes en vigueur, parmi lesquelles on peut citer HL7, ASTM et XML.

Les tubes comportent de préférence des moyens d'identification et de suivi par code barres, par puces électronique et/ou étiquette magnétique.

L'application du dispositif selon l'invention aux analyseurs sur sang total est nouvelle. Elle requiert l'intégration de moyens de conservation des sangs de contrôle, de mélange et de prélèvement de l'échantillon dans l'analyseur.

Sous un autre aspect, l'invention concerne un analyseur sanguin comprenant un dispositif tel que défini ci-dessus, selon la revendication 16.

Dans la description qui suit, faite seulement à titre d'exemple on se réfère aux dessins annexés, sur lesquels:
- la figure 1 est une vue en perspective d'un support de tubes en contact avec un bloc de refroidissement selon l'invention;
- la figure 2 est une vue illustrant de déplacement du support des tubes de la figure 1 dans leur mouvement d'agitation;
- les figures 3A et 3B sont respectivement des vues en perspective et de face d'un dispositif de contrôle de qualité intégré dans un analyseur fonctionnant en sang total selon l'invention;

- la figure 4 est un schéma illustrant les modes de fonctionnement du dispositif de contrôle de qualité selon l'invention; et
- la figure 5 est un schéma illustrant sous la forme d'un organigramme le processus d'interprétation et de validation des résultats mis en oeuvre par le dispositif selon l'invention.

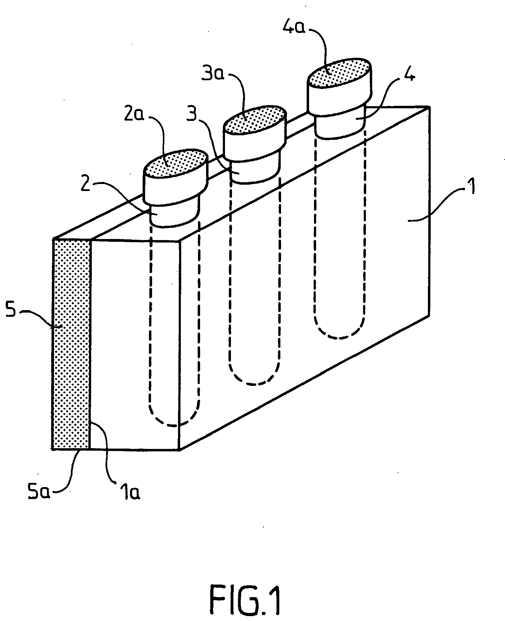

Le support de tubes en forme de parallélépipède rectangle, représenté avec la référence 1 sur la figure 1, comporte trois logements cylindriques d'axes longitudinaux parallèles entre eux dans lesquels sont rangés trois tubes de sangs de contrôle 2, 3, 4 fermés par des bouchons 2a, 3a, 4a. Les tubes 2, 3, 4 renfermant des sangs de contrôle, sont réfrigérés au travers du support 2 par un bloc de refroidissement 5. Les sangs contenus dans les tubes 2, 3, 4 peuvent correspondre aux niveaux bas, normal et haut de contrôle mais on peut imaginer qu'il y ait deux ou trois niveaux identiques ou que l'on utilise qu'un seul niveau.

Le bloc de refroidissement 5 en forme de parallélépipède rectangle est en contact étroit par une de ses faces 5a avec une face la en regard du support de tubes 1 lorsque le contrôle de qualité n'est pas utilisé, c'est à dire dans les conditions de repos, pour conserver les sangs à la température recommandée par le fournisseur.

De façon avantageuse, le bloc 5 peut être un bloc de refroidissement fonctionnant suivant le principe de l'effet Peltier qui permet de contrôler la température du bloc à l'aide de moyens de régulation électronique non représentés, le réchauffage des tubes ayant lieu par exemple en coupant le courant traversant le bloc de refroidissement. Cependant tout autre procédé de refroidissement peut également être utilisé. Ainsi il est possible aussi d'utiliser l'effet Peltier en sens inverse pour provoquer le réchauffage des tubes et donc la remise en température des sangs de contrôle.

Le support de tubes 1 est réalisé dans un métal ou tout autre matière conductrice de la température de façon à ce qu'il puisse rapidement prendre la température du bloc de refroidissement et donc réguler la température des sangs de contrôle à la même température que celle du bloc de refroidissement.

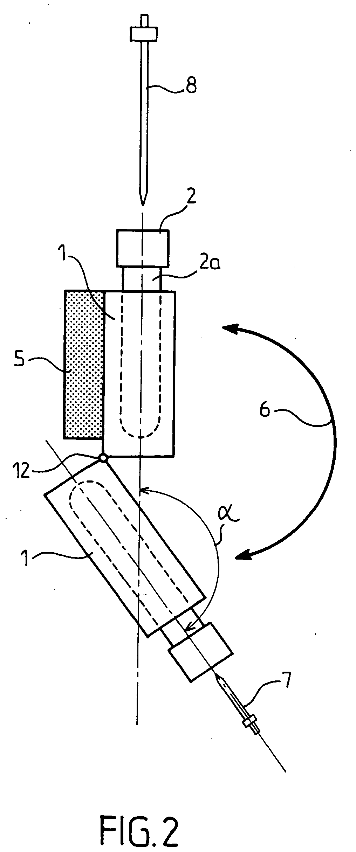

Comme le montre la figure 2, où les éléments homologues à ceux de la figure 1 sont représentés avec les mêmes références, l'agitation du sang est effectuée par retournement répété des tubes 2, 3, 4 autour d'une charnière 12, disposée sensiblement à l'horizontale, depuis une position haute pour laquelle les tubes sont orientés verticalement avec les bouchons 2a, 3a, 4a au dessus jusqu'à une position basse pour laquelle les tubes de sang 2, 3, 4 sont orientés verticalement mais présentent leurs bouchons 2a, 3a, 4a, vers le bas et vice versa. L'angle α de retournement peut être quelconque et compris par exemple entre 100° et 180°. Les moyens mécaniques permettant d'effectuer ce mouvement sont à la portée de l'homme de métier et ne sont pas représentés. Le mouvement alternatif représenté par la double flèche 6 est répété plusieurs fois afin d'obtenir une agitation du sang conforme aux spécifications données par le fournisseur des sangs de contrôle.

Dans cette réalisation préférentielle, on voit qu'il est possible d'effectuer le prélèvement du sang avec le tube en position haute par des moyens de prélèvement symbolisés par une aiguille 8 ou avec le tube en position basse par des moyens de prélèvement symbolisés par une aiguille 7.

On voit également que l'on peut obtenir une remise en température rapide des sangs de contrôle en désolidarisant le support de tube 1 du bloc de refroidissement 5 par une rotation de l'angle α autour de la charnière 12. Cette solution peut s'appliquer aussi à des blocs de refroidissement qui ne fonctionnent pas par effet Peltier.

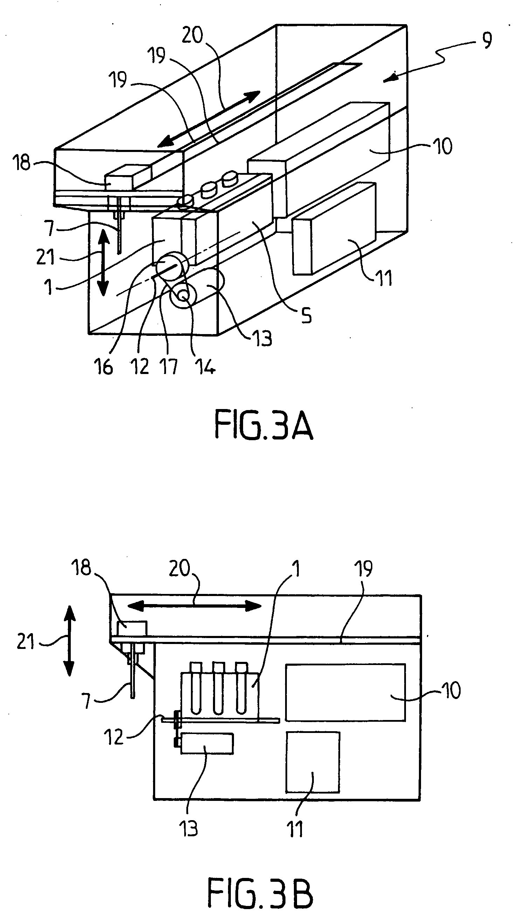

On se réfère maintenant aux figures 3A et 3B qui représentent un exemple d'intégration d'un dispositif de contrôle de qualité dans un instrument d'analyse sur sang total 9. L'exemple se rapporte à un compteur de cellules du type de celui décrit par exemple dans le brevet FR 97 13503 déposé au nom de la société Demanderesse.

Le dispositif de contrôle comprend un bloc support de tubes 1 en contact avec un bloc de refroidissement à effet Peltier 2, une aiguille de prélèvement 7, et un bloc de comptage 10 comportant des cuves de mélange et de rinçage (non représentées). On voit également sur les figures 3A et 3B le bloc d'alimentation électrique de l'instrument d'analyse 9.

Le bloc support de tubes 1 est entraîné en rotation par l'axe charnière 12 qui est couplé à un moteur 13 par l'intermédiaire d'un réducteur formé par une poulie d'entraînement 14 solidaire de l'axe du moteur 13 et par une poulie menée 16 solidaire de l'axe charnière 12, la poulie menée 16 étant entraînée par la poulie d'entraînement 14 par l'intermédiaire d'une courroie 17.

L'aiguille de prélèvement 7 est solidaire d'un chariot 18 mobile en translation sur deux rails 19 qui lui permettent de se déplacer d'un mouvement horizontal symbolisé par la double flèche 20, successivement au dessus du sang de patients à analyser, des sangs de contrôle contenus dans les tubes 4, 5, 6, puis du bloc de comptage 10 comportant des cuves de mélange et de rinçage. Le déplacement du chariot 18 peut être assuré par tout moyen connu non représenté, tel que par exemple un moteur électrique de type pas à pas, intégré au chariot 18 commandé par un microprocesseur.

L'aiguille de prélèvement 7 est également animée d'un mouvement vertical symbolisé par la double flèche 21 qui lui permet de descendre dans les tubes 2, 3, 4 afin d'effectuer le prélèvement du sang ou de descendre dans le bloc de comptage 10 comportant des cuves de mélange et de rinçage 10 pour effectuer des rinçages ou des dilutions de sang. Le déplacement de l'aiguille 7 peut être assuré par un moteur électrique de type pas à pas, non représenté, couplé par exemple à une crémaillère supportant l'aiguille 7.

Une porte d'accès non représentée permet le remplacement des tubes de contrôle lorsque ceux-ci sont vides ou atteignent la date de péremption.

La disposition des tubes de sang de contrôle n'est pas limitée à la seule représentation des figures 3A et 3B dans lesquelles les moyens d'agitation représentés ne sont donnés qu'à titre d'exemple. On pourra également utiliser des moyens d'agitation par Vortex à faible vitesse, balancement et/ou retournement des types précités dans l'art antérieur en leur adaptant des moyens de refroidissement nécessaires à la conservation et à la remise en température des tubes.

Les moyens de prélèvement peuvent nécessiter d'avoir un prélèvement fonctionnant avec le tube retourné, c'est à dire avec le bouchon en position basse. C'est souvent le cas avec les analyseurs utilisant une vanne d'échantillonnage. La rotation de l'ensemble des contrôles de qualité dans l'agitateur permet les deux configurations de perçage, le perçage pouvant être réalisé par le mouvement de l'aiguille 7 vers le contrôle de qualité ou par le mouvement d'une partie ou de la totalité de l'appareillage lié au contrôle de qualité vers l'aiguille 7.

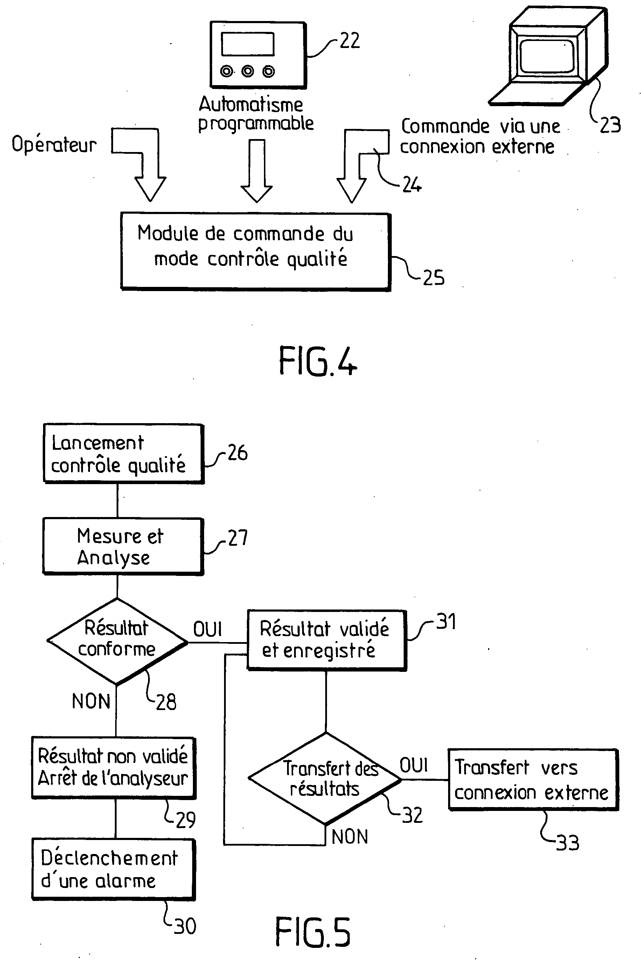

Comme on peut le voir sur la figure 4, le lancement du contrôle de qualité peut être effectué de trois façons différentes. Il peut être lancé soit par un opérateur, soit automatiquement à l'aide d'un automate programmable 22 suivant une programmation qui permet par exemple de lancer le contrôle de qualité le matin ou pendant la nuit, soit encore par une console 23 via un réseau Intranet ou Internet 24 mettant en oeuvre par exemple les technologies de réseau connues sous les désignations HL7, ASTM et XML afin de permettre à un laboratoire centrale dont dépend l'analyseur sang total, de lancer la procédure de contrôle de qualité. Ces trois modes de lancement sont gérés par l'intermédiaire d'un module 25 qui permet le déclenchement du mode contrôle de qualité.

L'algorithme représenté par les étapes 26 à 33 de l'organigramme de la figure 5 permet l'interprétation et la validation des résultats et la génération d'alarmes au cas où le contrôle de qualité est défaillant. La procédure commence à l'étape 26 par le lancement de la séquence de contrôle, ce qui implique la remise en température et l'agitation du contrôle de qualité. L'étape suivante 27 représente l'étape de mesure et d'analyse. A l'étape 28 une comparaison est effectuée entre les résultats de l'analyse et les valeurs attendues du contrôle de qualité. Si les résultats ne sont pas corrects, ceux-ci ne sont pas validés et l'étape 29 permet l'enregistrement des données, l'arrêt temporaire de l'analyseur 9 et le déclenchement d'une alarme à l'étape 30.

Si à l'étape 28 les résultats de l'analyse sont corrects, les résultats sont enregistrés à l'étape 31 et en fonction d'un test effectué sur les moyens de connexion à l'étape 32, les résultats sont envoyés vers un serveur via des fonctions de connexion externe 33.

Dans cette procédure, l'identification et le suivi du sang examiné est réalisé par tout moyen d'identification connu tel que : code barre, puce électronique et/ou étiquette magnétique accompagnant les tubes.

## Revendications

1. Dispositif de contrôle de qualité pour un analyseur sanguin (9) fonctionnant en sang total, **caractérisé en ce qu'**il comprend des moyens de conservation (1, 5) par refroidissement des sangs de contrôle, des moyens (1, 5) de remise en température des sangs de contrôle à la température prescrite par le fabricant des sangs de contrôle, des moyens d'agitation (1, 12, 13, 14, 16, 17) pour la remise en suspension des cellules et des moyens de prélèvement (7) du sang ainsi préparé, ce qui permet d'intégrer le dispositif à l'analyseur sanguin, **en ce que** les moyens de conservation (1, 5) des sangs de contrôle comprennent un nombre déterminé de tubes (2, 3, 4) fermés par un bouchon (2a, 3a, 4a) et rangés dans un support (1) en contact avec un bloc de refroidissement (5) permettant de réguler la température et de maintenir une température optimum pour la conservation du sang de contrôle, et **en ce que** le support de tubes (1) est désolidarisé du bloc de refroidissement (5) pour la remise en température des sangs de contrôle.

2. Dispositif selon la revendication 1, **caractérisé en ce que** le bloc de refroidissement (5) est un bloc de refroidissement à effet Peltier.

3. Dispositif selon la revendication 2, **caractérisé en ce que** le courant alimentant le bloc de refroidissement à effet Peltier (5) est coupé pendant une période de temps déterminée pour la remise en température des sangs de contrôle.

4. Dispositif selon la revendication 2, **caractérisé en ce que** le bloc de refroidissement à effet Peltier (5) est commandé pour remettre et maintenir le contrôle de qualité à sa température d'utilisation selon les spécifications du fabricant.

5. Dispositif selon l'une des revendications 1 à 4, **caractérisé en ce que** les moyens d'agitation (1, 12, 13, 14, 16, 17) sont des moyens d'agitation par balancement et/ou retournement formés par le support de tube (1) articulé autour d'une charnière (12) du bloc de refroidissement (2).

6. Dispositif selon la revendication 5, **caractérisé en ce que** l'angle de retournement des moyens de retournement est compris entre 100° et 180°.

7. Dispositif selon l'une des revendications 1 à 4, **caractérisé en ce que** les moyens d'agitation sont des moyens d'agitation par Vortex à faible vitesse.

8. Dispositif selon l'une des revendications 1 à 7, **caractérisé en ce que** les moyens de prélèvement du sang sont formés par une aiguille (7) propre à aspirer le sang dans les tubes.

9. Dispositif selon la revendication 8, **caractérisé en ce que** l'aiguille (7) est animée d'un mouvement transversal au dessus des tubes de sang à analyser et des sangs de contrôle (2, 3, 4) ainsi qu'au dessus d'un bloc de comptage (10) comportant des cuves de mélange et de rinçage et d'un mouvement vertical pour pénétrer dans les tubes par perçage des bouchons ou de descendre dans le bloc de comptage (10) comportant des cuves de mélange et de rinçage pour effectuer les rinçages ou les dilutions du sang.

10. Dispositif selon la revendication 9, **caractérisé en ce que** le perçage des bouchons (2a, 3a, 4a) s'effectue lorsque les tubes sur leur support (1) sont en position haute ou basse.

11. Dispositif selon l'une des revendications 1 à 10, **caractérisé en ce qu'**il comprend des moyens de traitement programmables (25,...,33) pour vérifier que les valeurs obtenues en passant chaque contrôle de qualité correspondent aux valeurs limites et attendues du sang de contrôle.

12. Dispositif selon la revendication 11, **caractérisé en ce que** les moyens de traitement déclenchent une alarme (30) si les valeurs obtenues lors du passage du contrôle de qualité sont en dehors des limites attendues.

13. Dispositif selon l'une des revendications 1 à 12, **caractérisé en ce qu'**il comprend des moyens (23) pour déclencher la procédure de contrôle de qualité soit directement par un opérateur ou de manière automatique (22) ou par une connexion externe vers un module de commande (25).

14. Dispositif selon l'une des revendications 1 à 13, caractérisé en ce le transfert et l'analyse des données s'effectuent par l'intermédiaire d'un réseau interne ou externe mettant en oeuvre les normes en vigueur, parmi lesquelles on peut citer HL7, ASTM et XML.

15. Dispositif selon l'une des revendications 1 à 14, **caractérisé en ce que** les tubes (2, 3, 4) comportent des moyens d'identification et de suivi par code barres, par puces électronique et/ou étiquette magnétique.

16. Analyseur sanguin comprenant un dispositif selon l'une des revendications 1 à 15, intégré à l'analyseur.

## Patentansprüche

1. Qualitätskontrollvorrichtung für ein Blutanalysegerät (9) unter Verwendung von Vollblut, **dadurch gekennzeichnet, dass** sie Mittel zur Konservierung (1, 5) durch Kühlung des Kontrollblutes, Mittel (1, 5) zur Wiederherstellung der Temperatur des Kontrollblutes auf die vom Hersteller des Kontrollblutes vorgeschriebene Temperatur, Rührmittel (1, 12, 13, 14, 16, 17) zum Resuspendieren der Zellen und Mittel (7) zur Entnahme des so hergestellten Blutes umfasst, wodurch eine Integration der Vorrichtung in das Blutanalysegerät ermöglicht wird, dadurch, dass die Mittel zur Konservierung (1, 5) des Kontrollblutes eine festgelegte Anzahl an Röhrchen (2, 3, 4) umfassen, die durch einen Stopfen (2a, 3a, 4a) verschlossen und in einem Träger (1) in Kontakt mit einem Kühlblock (5) angeordnet sind, der es ermöglicht, die Temperatur zu regeln und eine optimale Temperatur für die Konservierung des Kontrollblutes aufrechtzuerhalten, und dadurch, dass der Röhrchenträger (1) vom Kühlblock (5) getrennt ist, um die Temperatur des Kontrollblutes wiederherzustellen.

2. Vorrichtung nach Anspruch 1, **dadurch gekennzeichnet, dass** der Kühlblock (5) ein Peltier-Kühlblock ist.

3. Vorrichtung nach Anspruch 2, **dadurch gekennzeichnet, dass** der den Peltier-Kühlblock (5) speisende Strom zur Wiederherstellung der Temperatur des Kontrollblutes für eine festgelegte Zeitspanne abgeschaltet wird.

4. Vorrichtung nach Anspruch 2, **dadurch gekennzeichnet, dass** der Peltier-Kühlblock (5) so gesteuert wird, dass die Qualitätskontrolle bei ihrer Betriebstemperatur gemäß den Herstellerspezifikationen wiederhergestellt und aufrechterhalten wird.

5. Vorrichtung nach einem der Ansprüche 1 bis 4, **dadurch gekennzeichnet, dass** die Rührmittel (1, 12, 13, 14, 16, 17) mittels Schwenken und/oder Drehen, gebildet durch den Röhrchenträger (1), der um ein Scharnier (12) des Kühlblocks (2) gelenkig angebracht ist, rührende Mittel sind.

6. Vorrichtung nach Anspruch 5, **dadurch gekennzeichnet, dass** der Drehwinkel der Drehmittel zwischen 100° und 180° liegt.

7. Vorrichtung nach einem der Ansprüche 1 bis 4, **dadurch gekennzeichnet, dass** die Rührmittel durch Wirbel bei niedriger Drehzahl rührende Mittel sind.

8. Vorrichtung nach einem der Ansprüche 1 bis 7, **dadurch gekennzeichnet, dass** die Blutentnahmemittel durch eine Nadel (7) ausgebildet sind, die geeignet ist, Blut in die Röhrchen zu ziehen.

9. Vorrichtung nach Anspruch 8, **dadurch gekennzeichnet, dass** die Nadel (7) zu einer Querbewegung über den Röhrchen mit dem zu analysierenden Blut und dem Kontrollblut (2, 3, 4) sowie über einem Zählerblock (10) mit Misch- und Spülbehältern angetrieben wird, und einer Vertikalbewegung, um in die Röhrchen durch Durchstechen der Stopfen einzudringen oder in den Zählerblock (10) mit Misch- und Spülbehältern zum Durchführen von Spülungen oder Verdünnungen des Blutes hinabzusteigen.

10. Vorrichtung nach Anspruch 9, **dadurch gekennzeichnet, dass** das Durchstechen der Stopfen (2a, 3a, 4a) erfolgt, wenn sich die Röhrchen auf ihrem Träger (1) in der oberen oder unteren Position befinden.

11. Vorrichtung nach einem der Ansprüche 1 bis 10, **dadurch gekennzeichnet, dass** sie programmierbare Verarbeitungsmittel (25, ...,33) zum Überprüfen, ob die durch die Durchführung jeder Qualitätskontrolle erhaltenen Werte den Grenzwerten und erwarteten Werten des Kontrollblutes entsprechen, umfasst.

12. Vorrichtung nach Anspruch 11, **dadurch gekennzeichnet, dass** die Verarbeitungsmittel einen Alarm (30) auslösen, wenn die bei der Qualitätskontrolle erhaltenen Werte außerhalb der erwarteten Grenzen liegen.

13. Vorrichtung nach einem der Ansprüche 1 bis 12, **dadurch gekennzeichnet, dass** sie Mittel (23) zum Einleiten des Qualitätskontrollverfahrens entweder direkt durch einen Bediener oder automatisch (22) oder durch eine externe Verbindung zu einem Steuermodul (25) umfasst.

14. Vorrichtung nach einem der Ansprüche 1 bis 13, **dadurch gekennzeichnet, dass** die Datenübertragung und -analyse über ein internes oder externes Netzwerk erfolgt, das die geltenden Normen, darunter HL7, ASTM und XML, umsetzt.

15. Vorrichtung nach einem der Ansprüche 1 bis 14, **dadurch gekennzeichnet, dass** die Röhrchen (2, 3, 4) Identifikations- und Nachverfolgungsmittel mittels Barcode, elektronischer Chips und/oder magnetischem Etikett umfassen.

16. Blutanalysegerät, umfassend eine Vorrichtung nach einem der Ansprüche 1 bis 15, die in das Analysegerät integriert ist.

## Claims

1. Quality control device for a blood analyser (9) using whole blood, **characterised in that** it comprises means (1, 5) of storage by refrigeration for control bloods, means (1, 5) of restoring to temperature of the control bloods to the temperature prescribed by the manufacturer of the control bloods, stirring means (1, 12, 13, 14, 16, 17) for resuspension of the cells, and sampling means (7) of the blood thus prepared, which makes it possible to incorporate the device in the blood analyser, **in that** the means (1, 5) of storing control bloods comprise a specified number of tubes (2, 3, 4) sealed by a bung (2a, 3a, 4a) and arranged in a support (1) in contact with a refrigeration block (5) making it possible to adjust the temperature and to maintain an optimum temperature for storing the control blood, and **in that** the tube support (1) is disconnected from the refrigeration block (5) for restoring the temperature of the control bloods.

2. Device according to claim 1, **characterised in that** the refrigeration block (5) is a Peltier effect refrigeration block.

3. Device according to claim 2, **characterised in that** the current supplying the Peltier effect refrigeration block (5) is interrupted for a specified period of time for restoration of the temperature of the control bloods.

4. Device according to claim 2, **characterised in that** the Peltier effect refrigeration block (5) is controlled in order to reset and maintain the quality control to its utilisation temperature according to the specifications of the manufacturer.

5. Device according to one of claims 1 to 4, **characterised in that** the stirring means (1, 12, 13, 14, 16, 17) are stirring means operating by rocking and/or inversion formed by the tube support (1) articulated about a hinge (12) of the refrigeration block (2).

6. Device according to claim 5, **characterised in that** the angle of inversion of the inversion means is between 100° and 180°.

7. Device according to one of claims 1 to 4, **characterised in that** the stirring means are low-speed Vortex stirring means.

8. Device according to one of claims 1 to 7, **characterised in that** the blood sampling means are formed by a needle (7) capable of drawing the blood from the tubes.

9. Device according to claim 8, **characterised in that** the needle (7) is driven in a transverse movement over the tubes of blood to be analysed and the control bloods (2, 3, 4) as well as over a counting block (10) comprising mixing and rinsing tanks and in a vertical movement in order to penetrate into the tubes by piercing the bungs or by descending into the counting block (10) comprising mixing and rinsing tanks in order to carry out rinsing or dilutions of the blood.

10. Device according to claim 9, **characterised in that** the piercing of the bungs (2a, 3a, 4a) is effected when the tubes on their support (1) are in a high or low position.

11. Device according to one of claims 1 to 10, **characterised in that** it comprises programmable processing means (25,...,33) for checking that the values obtained by passing through each quality control correspond to the limit values and the values expected of the control blood.

12. Device according to claim 11, **characterised in that** the processing means trigger an alarm (30) if the values obtained during running of the quality control are outside the expected limits.

13. Device according to one of claims 1 to 12, **characterised in that** it comprises means (23) of triggering the quality control procedure either directly by an operator or automatically (22) or via an external connection to a control unit (25).

14. Device according to one of claims 1 to 13, **characterised in that** the transfer and analysis of the data are effected via an internal or external network implementing the standards currently in force, among which can be cited HL7, ASTM and XML.

15. Device according to one of claims 1 to 14, **characterised in that** the tubes (2, 3, 4) have means of identification and tracking by barcodes, electronic chips and/or magnetic label.

16. Blood analyser comprising a device according to one of claims 1 to 15, incorporated in the analyser.
